# EUROPÄISCHE PATENTANMELDUNG

(11) **EP 1 974 669 A2**
(43) Veröffentlichungstag der Anmeldung: **01.10.2008**
(21) Anmeldenummer: 08004748.3
(22) Anmeldetag: 14.03.2008
(51) Int. Cl.: A61B 8/00

(54) **Vorrichtung zur Ortung einer in einen Körper eingestochenen Kanüle**

(30) Priorität: 29.03.2007 DE 102007015553
(71) Anmelder: Pajunk GmbH & Co. KG Besitzverwaltung, 78187 Geisingen (DE)
(72) Erfinder: Pajunk, Heinrich, 78187 Geisingen (DE); Pajunk, Horst, 78187 Geisingen (DE); Tsui, Ban C. H., Alberta T6G 2G3 (CA)
(74) Vertreter: Mussgnug, Bernd

(57) **Zusammenfassung**

Zur Ortung einer in einen Körper eingestochenen Kanüle (10) mittels eines Ultraschallkopfes wird auf dem proximalen Ende der Kanüle (10) mittels eines Adapters (14) ein Lichtzeiger (16) angebracht. Der Lichtzeiger (16) sendet einen zur Kanüle (10) achsparallelen Lichtstrahl (18) aus. Beim Einstechen der Kanüle (10) erzeugt der Lichtstrahl (18) auf der Körperoberfläche eine Lichtmarke, auf welcher der Ultraschallkopf aufgesetzt wird.

## Beschreibung

Die Erfindung betrifft eine Vorrichtung zur Ortung einer in einen Körper eingestochenen Kanüle gemäß dem Oberbegriff des Patentanspruchs 1.

In der Medizin werden Kanülen zu unterschiedlichen Anwendungen in den menschlichen Körper eingestochen. Insbesondere werden solche Kanülen in der Anästhesie verwendet, z. B. in der Plexus- und Spinal-Anästhesie. Hierbei ist es äußerst wichtig, die Position der Kanüle, insbesondere der Kanülenspitze im Körper zu lokalisieren und zu verfolgen. Hierzu ist es bekannt, auf die Körperoberfläche einen Ultraschallkopf aufzusetzen, mit welchem die Kanüle geortet werden kann.

In der Praxis wird der Ultraschallkopf im Allgemeinen von Hand geführt aufgesetzt. Dabei können Schwierigkeiten bei der Ortung der Kanüle auftreten, wenn der Ultraschallkopf so aufgesetzt wird, dass sein Sichtfeld nicht den Einstichkanal der Kanüle erfasst.

Der Erfindung liegt die Aufgabe zugrunde, eine Vorrichtung zur Ortung einer in den Körper eingestochenen Kanüle mittels eines Ultraschallkopfes zu schaffen, durch welche zuverlässig gewährleistet ist, dass das Ultraschallbild die in den Körper eindringende Kanüle erfasst.

Diese Aufgabe wird erfindungsgemäß gelöst durch eine Vorrichtung mit den Merkmalen des Patentanspruchs 1.

Vorteilhafte Ausführungen der Erfindung sind in den Unteransprüchen angegeben.

Erfindungsgemäß wird auf die Kanüle ein Lichtzeiger, vorzugsweise ein Laserpointer aufgesetzt, der einen Lichtstrahl in distaler Richtung in einer die Längsachse der Kanüle einschließenden Ebene aussendet. Vorzugsweise verläuft der Lichtstrahl im Wesentlichen achsparallel zur Kanüle. Beim Einstechen der Kanüle erzeugt der Lichtzeiger auf der Körperoberfläche distal vor der Einstichstelle eine Lichtmarke. Diese Lichtmarke gibt die Stelle an, an welcher der handgeführte Ultraschallkopf aufgesetzt werden muss. Da der Lichtstrahl und die Kanüle in derselben axialen Ebene liegen, ist gewährleistet, dass das Ultraschall-Sichtfeld des Ultraschallkopfes die Kanülenachse und damit die in den Körper eindringende Kanüle erfasst.

Der Lichtzeiger, insbesondere in der Form eines Laserpointers, wie er vielfach bei Präsentationen, als Schlüsselanhänger und dergleichen eingesetzt wird, ist ein kostengünstiger Artikel. Vorzugsweise wird der Lichtzeiger mittels eines Adapters lösbar an der Kanüle, insbesondere an einem proximalen Ansatz der Kanüle angebracht. Dadurch ist eine kostengünstige Vielfach-Verwendung des Lichtzeigers möglich.

Die von dem Lichtzeiger erzeugte Lichtmarke kann eine beliebige Form aufweisen, im einfachsten Falle die Form eines Lichtpunktes. Vorteilhaft ist es, wenn die Lichtmarke eine zweidimensionale Form mit in unterschiedlichen Richtungen verlaufenden Strukturen aufweist. Zweckmäßig ist z. B. die Form eines Kreuzes, wobei ein Arm des Kreuzes in der Kanülenrichtung und ein zweiter Arm des Kreuzes quer zur Kanülenrichtung verläuft. Durch diese Ausbildung der Lichtmarke wird die Zielgenauigkeit beim Aufsetzen des Ultraschallkopfes zusätzlich erhöht. Die zweidimensionale, insbesondere kreuzförmige Ausbildung der Lichtmarke lässt auch eine Rotation der Kanüle mit dem aufgesetzten Lichtzeiger leicht erkennen. Es kann dadurch sichergestellt werden, dass die durch den Lichtstrahl des Lichtzeigers und die Kanüle aufgespannte Ebene senkrecht zur Körperoberfläche verläuft und damit der Ultraschallkopf exakt in dieser Ebene aufgesetzt wird.

Im Folgenden wird die Erfindung anhand eines in der Zeichnung dargestellten Ausführungsbeispieles näher erläutert. Es zeigen:
- Figur 1: eine Seitenansicht einer Kanüle mit aufgesetztem Lichtzeiger und
- Figur 2: die geometrischen Verhältnisse beim Einstich der Kanüle.

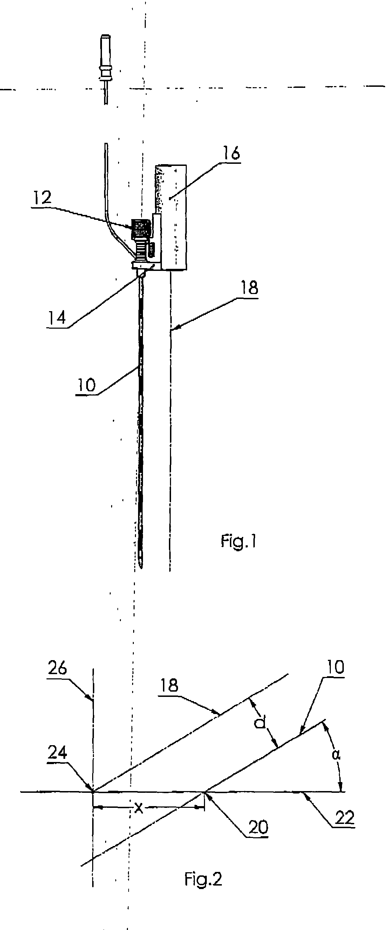

In Figur 1 ist die erfindungsgemäße Vorrichtung in Verbindung mit einer handelsüblichen Kanüle, z. B. einer Tuohy-Kanüle gezeigt.

Die Kanüle 10 weist an ihrem proximalen Ende einen Ansatz 12 auf. An diesem Ansatz 12 wird lösbar ein Adapter 14 befestigt. Der Adapter 14 trägt einen Lichtzeiger 16, der insbesondere als Laserpointer ausgebildet ist. Solche Laserpointer sind allgemein bekannter Stand der Technik, so dass eine detaillierte Beschreibung überflüssig ist. Insbesondere weist ein solcher Laserpointer eine batteriegespeiste Laserdiode auf, die Licht im sichtbaren Wellenlängenbereich emittiert. Aus Kostengründen werden rote Laserdioden bevorzugt.

Der Adapter 14 ist vorzugsweise aus Kunststoff gefertigt und kann auf den Ansatz 12 der Kanüle 10 geklemmt werden, z. B. durch federbelastete Klemmbacken oder mittels einer Schraubklemmung. Mit dem Lichtzeiger 16 kann der Adapter 14 fest verbunden sein.

Der Lichtzeiger 16 sendet einen Lichtstrahl 18 aus, der in Figur 1 gestrichelt eingezeichnet ist. Der Lichtstrahl 18 verläuft achsparallel zu der Mittelachse der Kanüle 10. Vorzugsweise ist der Lichtzeiger 16 durch eine Austrittsblende so gestaltet, dass der Lichtstrahl 18 eine kreuzförmige Lichtmarke erzeugt, deren einer Arm in der durch den Lichtstrahl 18 und die Kanüle 19 aufgespannten Ebene liegt und deren anderer Arm senkrecht zu dieser Ebene verläuft.

Wie in Figur 2 schematisch gezeigt ist, wird die Kanüle 10 an einem Einstichpunkt 20 unter einem Winkel α in die Körperoberfläche 22 des Patienten eingestochen. Die Kanüle 10 mit dem Lichtzeiger 16 wird dabei so geführt, dass die von der Kanülenachse und dem Lichtstrahl 18 aufgespannte Ebene (in Figur 2 ist dies die Zeichenebene) zur Ebene der Körperoberfläche senkrecht liegt. Der Lichtzeiger 16 ist an der Kanüle 10 so angebracht, dass der von dem Lichtzeiger 16 ausgesandte Lichtstrahl 18 einen Abstand d von der Kanülenachse aufweist. Dementsprechend erzeugt der Lichtstrahl 18 eine Lichtmarke in einem Punkt 24, der in einem Abstand x distal vor dem Einstichpunkt 20 der Kanüle 10 liegt. Der Abstand x beträgt dabei d/sin α. Je flacher die Kanüle 10 eingestochen wird, um so größer wird somit der Abstand x. Bei einem Abstand d zwischen der Kanüle 10 und dem Lichtstrahl 18 von beispielsweise d=17mm ergeben sich Abstände x zwischen dem Einstichpunkt 20 und der Lichtmarke 24 von etwa 31mm bei einem Einstichwinkel α = 30°, von etwa 42mm bei einem Einstichwinkel α = 20° und von etwa 65mm bei einem Einstichwinkel α = 15°.

Auf den Punkt 24 der Lichtmarke, z. B. auf dem Kreuzungspunkt der kreuzförmigen Lichtmarke wird ein in der Zeichnung nicht dargestellter an sich bekannter handgeführter Ultraschallkopf aufgesetzt, wobei die in Figur 2 strichpunktierte Achse 26 seines Sichtfeldes senkrecht zur Körperoberfläche 22 verläuft. Dadurch ist gewährleistet, dass das Sichtfeld des Ultraschallkopfes die Kanüle 10 bei deren Eindringen in den Körper entlang der Figur 2 gestrichelt gezeichneten Linie erfasst.

## Patentansprüche

1. Vorrichtung zur Ortung einer in einen Körper eingestochenen Kanüle (10) mittels eines auf die Körperoberfläche (22) aufsetzbaren Ultraschallkopfes,
**gekennzeichnet durch** einen proximal an der Kanüle (10) angeordneten Lichtzeiger (16), der einen Lichtstrahl (18) in distaler Richtung in einer die Längsachse der Kanüle (10) einschließenden Ebene aussendet und auf der Körperoberfläche (22) distal vor der Einstichstelle (20) der Kanüle (10) eine Lichtmarke (24) für das Aufsetzen des Ultraschallkopfes erzeugt.

2. Vorrichtung nach Anspruch 1,
**dadurch gekennzeichnet, dass** der Lichtzeiger (16) ein Laserpointer ist.

3. Vorrichtung nach Anspruch 1 oder 2,
**dadurch gekennzeichnet, dass** der Lichtzeiger (16) mittels eines Adapters (14) lösbar an einem proximalen Ansatz (12) der Kanüle (10) befestigbar ist.

4. Vorrichtung nach einem der vorhergehenden Ansprüche,
**dadurch gekennzeichnet, dass** der Lichtstrahl (18) des Lichtzeigers (16) achsparallel zur Längsachse der Kanüle (10) verläuft.

5. Vorrichtung nach einem der vorhergehenden Ansprüche,
**dadurch gekennzeichnet, dass** die Lichtmarke (24) eine zweidimensionale Form aufweist.

6. Vorrichtung nach Anspruch 5,
**dadurch gekennzeichnet, dass** die Lichtmarke (24) die Form eines Kreuzes aufweist, insbesondere mit einem die Achse der Kanüle (10) schneidenden Arm und einem zu diesem quer verlaufenden Arm.
